Europäisches Patentamt

European Patent Office

Office européen des brevets

(11) Numéro de publication: **0 253 697**
**A2**

# (12) DEMANDE DE BREVET EUROPEEN

(21) Numéro de dépôt: **87401399.8**

(51) Int. Cl.⁴: **A 61 K 31/195**

(22) Date de dépôt: **19.06.87**

(30) Priorité: **19.06.86 FR 8608872**

(43) Date de publication de la demande:
**20.01.88 Bulletin 88/03**

(84) Etats contractants désignés:
**AT BE CH DE ES FR GB GR IT LI LU NL SE**

(71) Demandeur: **INSTITUT NATIONAL DE LA SANTÉ ET DE LA RECHERCHE MEDICALE (INSERM)**
**101, rue de Tolbiac**
**F-75654 Paris Cedex 13 (FR)**

(72) Inventeur: **Vila, Jorge**
**43 Avenue Rockfeller**
**F-69003 Lyon (FR)**

**Thomasset, Nicole**
**43 rue du Tonkin**
**F-69100 Villeurbanne (FR)**

**Epstein, Alberto**
**403 Avenue du 8 mai 1945**
**F-69300 Caluire (FR)**

**Wild, Fabian**
**27 rue Guilloud**
**F-69003 Lyon (FR)**

(74) Mandataire: **Phélip, Bruno et al**
**c/o Cabinet Harlé & Phélip 21, rue de la Rochefoucauld**
**F-75009 Paris (FR)**

(54) **Médicament et composition médicamenteuse pour le traitement de maladies infectieuses dues aux virus, ainsi que pour le traitement des tumeurs.**

(57) Ce médicament est le L-glutamique acide γ monohydroxamate (GAH). Il inhibe de façon totale la multiplication virale (il appartiendrait à la catégorie des inducteurs de l'état cellulaire antiviral), et il montre une activité cytotoxique sur différentes lignées de tumeurs humaines (aussi bien tumeurs solides que lymphomes), la sélectivité d'action (cytolytique et irréversible sur cellules tumorales, et cytostatique réversible sur cellules normales) laissant à penser qu'il utilise une différence métabolique entre cellule tumorale et cellule normale. La composition médicamenteuse correspondante se présente sous la forme d'une poudre lyophilisée, constituée par le principe actif (GAH), à dissoudre extemporanément dans une solution physiologique, en vue de l'injection.

**Description**

## MEDICAMENT ET COMPOSITION MEDICAMENTEUSE POUR LE TRAITEMENT DE MALADIES INFECTIEUSES DUES AUX VIRUS, AINSI QUE POUR LE TRAITEMENT DES TUMEURS.

La présente invention se rapporte à un médicament, ainsi qu'à une composition médicamenteuse, permettant, d'une part, de traiter les maladies infectieuses dues aux virus, et, d'autre part, de traiter les tumeurs malignes. Ce médicament est le L-glutamique acide $\gamma$ monohydroxamate (GAH), de formule :

$$HOOC-(NH_2)CH-(CH_2)_2-CONHOH$$

L'acide glutamique monohydroxamate est typiquement formé par la réaction d'un groupe acyle activé avec l'hydroxylamine suivant la réaction :

$$HOOC-(NH_2)CH-(CH_2)_2-COX + NH_2OH \rightarrow HOOC-(NH_2)_2CH-(CH_2)_2-CONHOH + HX$$ , COX pouvant être un acide, amine, ester, anhydride.

Ce composé peut aussi être préparé par voie enzymatique à partir de l'acide L-glutamique, avec la glutamine synthétase.

On exposera ci-après, tout d'abord son activité antivirale et ensuite, son activité antitumorale.

## I - ACTIVITE ANTIVIRALE DU L'GLUTAMIQUE ACIDE $\gamma$ MONOHYDROXAMATE

Bien que des progrès aient été accomplis en ce qui concerne la lutte contre les infections virales, on ne dispose pas encore d'une gamme d'agents antiviraux aussi étendue, sûre et efficace que le sont, par exemple, les antibiotiques.

De par leur mode d'action, les agents antiviraux peuvent être classés, d'une façon large, en trois catégories, les frontières entre celles-ci étant souvent arbitraires :

- La première catégorie, à savoir celle des agents chimiques, constitue le groupe le plus étendu et celui qui se développe le plus vite. Etant donné que les virus sont des parasites intra-cellulaires obligés, l'efficacité de ces agents est déterminée par leur capacité à différencier le métabolisme viral du métabolisme cellulaire, autrement dit, le métabolisme de la cellule infectée et celui de la cellule non infectée. En d'autres termes, la caractéristique essentielle de ce groupe d'agents est leur spécificité d'action. Celle-ci détermine en même temps la force et la faiblesse de ces agents, car s'ils sont souvent très efficaces, du fait qu'ils peuvent inhiber spécifiquement une enzyme virale, leur spectre d'action est généralement très limité et ils finissent souvent par sélectionner des variants viraux résistants, de la même manière que le font les antibiotiques avec les bactéries (De Clercq E., Biochem. J. 205 : 1-13, 1982 ; Becker Y. et Hadar J., Prog. Med. Virol. 26 : 1-44, 1980).

- La deuxième catégorie est constituée par les stimulateurs du système immun. Mis à part les différents types de vaccins, ce groupe est constitué par des agents capables d'activer les défenses non spécifiques (macrophages, cellules "tueuses" naturelles) ou, plus rarement, les défenses spécifiques (lymphocytes "B" ou "T") de l'organisme. Pour la plupart, ces agents sont encore en phase expérimentale. Contrairement aux agents chimiques, les caractéristiques essentielles de leur mode d'action sont leur spectre d'action très large, et le fait qu'ils ne peuvent pas sélectionner de variants résistants. Cependant, ils sont, pour l'instant, en nombre limité et peu efficaces, bien que des progrès aient été récemment accomplis dans ce sens (Koff W.C., Showalter S.D., Hampar B. et Fidler I.J., Science 228 : 495-496, 1985).

- La troisième catégorie est celle des inducteurs de l'état cellulaire antiviral, groupe qui comprend l'interféron (IFN), les inducteurs d'IFN et les substituts d'IFN. Il s'agit d'un groupe encore très réduit de substances qui se caractérisent comme éveillant dans les cellules un état de résistance à l'infection virale. Ces agents cumulent la puissance de l'action au niveau cellulaire des agents chimiques et le spectre d'action très large des activateurs du système immun. Du fait qu'ils agissent sur toutes les cellules, qu'elles aient été infectées ou non, un élément déterminant de leur efficacité est leur faible pouvoir toxique envers les cellules.

Selon la présente invention, on a découvert que le L-glutamique acide $\gamma$ monohydroxamate (GAH) inhibe de façon totale la multiplication virale. On peut penser que cette substance appartient à la troisième catégorie sus-indiquée des agents antiviraux, bien que son mode précis d'action n'ait pas encore été totalement élucidé.

- Une expérience in vitro a permis de mettre en évidence que le L-glutamique acide $\gamma$ monohydroxamate provoque une inhibition très significative de deux virus pourtant très éloignés à plusieurs égards, l'un étant un virus à ARN (rougeole) et l'autre, un virus à ADN (HSV. 1) :

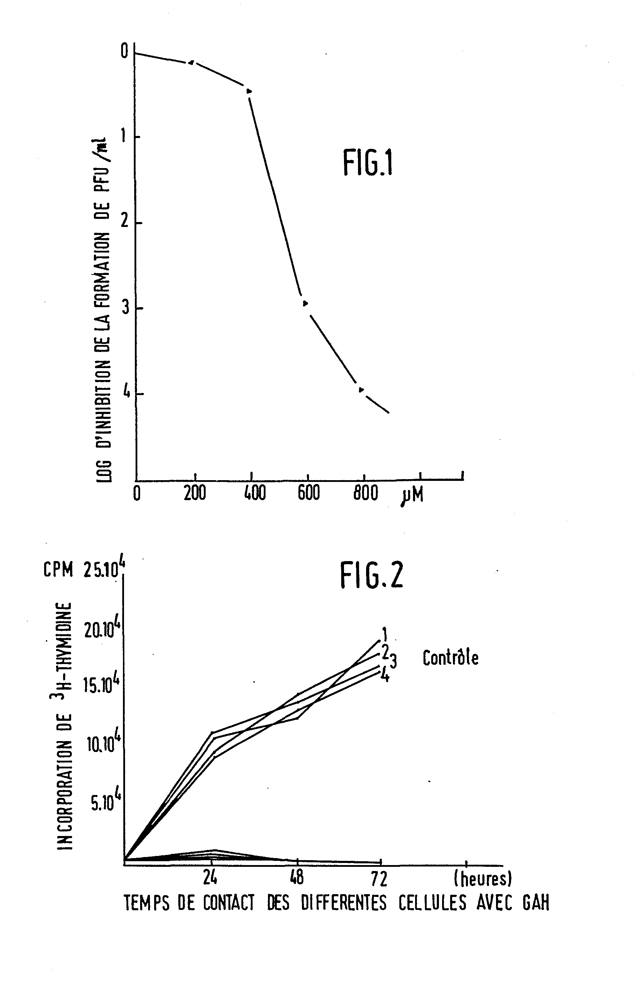

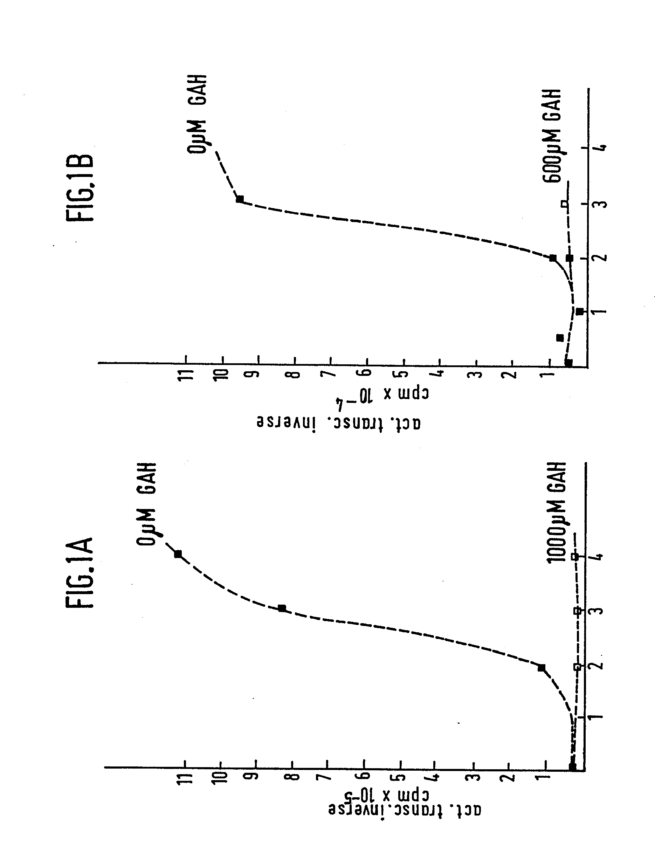

Des cellules VERO (lignée de cellules "normales") ont été infectées par le virus de l'herpès simplex du type 1 (HSV.1) ou par le virus de la rougeole à une multiplicité d'infection d'une particule infectieuse par cellule. Les cellules infectées ont été traitées par GAH aux doses indiquées sur la Figure 1. L'inhibition observée, emprimée en logarithme d'inhibition de la formation de plages de lyse cellulaire par le virus (PFU-"Plage Forming Unit") par ml de milieu, semble apparaître brusquement après avoir dépassé une concentration "seuil" située autour de 600 $\mu m$. par litre de milieu cellulaire. Le fait que la concentration optimale soit la même pour les deux virus testés, qui, comme indiqué ci-dessus, sont très différents, est en faveur d'une action de la substance au niveau cellulaire, action capable d'éveiller un état de résistance envers les virus infectants.

- D'autres expériences, qui ont été effectuées, montrent, les unes que l'inhibition observées augmente avec le temps de prétraitement, et les autres, que l'inhibition observée est indépendante de la

multiplicité d'infection, au moins jusqu'à une multiplicité de 10 particules infectieuses par cellule.

Le niveau d'inhibition maximal observé est égal, sinon supérieur, à celui obtenu avec les meilleurs inhibiteurs de la multiplication virale dont on dispose aujourd'hui. Le faible taux, voire l'absence, de toxicité du GAH pour les cellules (infectées ou non) mérite également d'être souligné.

Il a été également montré que le GAH provoque aussi une importante inhibition de la multiplication des virus appartenant à la famille des rétrovirus (virus à ARN possédant une activité dite de transcriptase inverse, c'est-à-dire une ADN polymérase-ARN dépendante). Ceci a été démontré avec le virus du sarcome de Rous (RSV, virus oncogène, sous-famille oncovirinae) et avec le virus visna (virus lytique, sous-famille lentivirinae à laquelle appartient aussi le H.I.V.).

1 - Des fibroblastes de poulet, en passage secondaire, sont ensemencés et traités, ou non, par le GAH à diverses concentrations. Ensuite, les cellules sont infectées par le RSV (souche B77). Le milieu de culture des cellules infectées est recueilli à différents temps post-infection et l'activité transcriptase inverse a été mesurée, cette mesure étant considérée généralement comme un reflet de la libération des particules virales dans les milieux de culture. La figure 1A, qui représente la courbe de l'activité transcriptase inverse (comptage de la radioactivité correspondante en coups par minute (CPM) en fonction du temps (en jours), montre qu'en présence de GAH (1mM) au moment de l'infection, l'activité transcriptase inverse dans le milieu de culture est indétectable, tandis que dans les contrôles non traités par le GAH, elle augmente de façon très importante.

En même temps, dans les cultures traitées par le GAH, aucune formation de foyers de cellules transformées par le RSV n'est observée, contrairement aux cultures témoins non traitées, où des foyers apparaissent au bout de trois jours d'infection.

2 - Des fibroblastes d'embryon de mouton sont traités à des doses variables par le GAH et sont ensuite infectés par le virus Visna. Comme dans le cas du RSV, la présence du GAH au moment de l'infection conduit à une diminution considérable de l'activité transcriptase inverse, par rapport aux cellules non traitées, comme le montre la figure 1B (même légende que la figure 1A).

Il est intéressant de signaler que, comme dans le cas des hespesvirus, l'activité antivirable apparaît à la même dose de GAH capable de provoquer l'arrêt de la prolifération cellulaire, ce qui est en accord avec la notion d'une action du GAH agissant en premier lieu au niveau cellulaire.

## II - ACTIVITE ANTITUMORALE DU L-GLUTAMIQUE ACIDE γ MONOHYDROXAMATE

Depuis une vingtaine d'années, il a été montré le rôle essentiel de la glutamine comme source d'énergie pour les cellules tumorales, alors que, dans les cellules normales, le glucose est principalement utilisé. Cette utilisation importante de la glutamine par les cellules cancéreuses a même pu être reliée au degré de malignité, étant donné qu'on sait déjà qu'il existe une bonne corrélation entre le degré de malignité des cellules d'hépatome et l'activité de la glutaminase. Ce rôle essentiel de la glutamine tient à sa participation dans les voies de bio-synthèse des protéines, des purines, des pyrimidines et du métabolisme énergétique.

La chimiothérapie s'est tournée vers l'utilisation d'analogues d'acides aminés comme agents anticancéreux pour mimer l'effet de l'acide aminé tout en espérant bloquer son action. La glutamine s'est trouvée un acide aminé de choix, du fait que son importante utilisation par la cellule tumorale et la diversité de ses voies d'utilisation intracellulaire. De nombreux analogues de cet acide aminé ont passé le stade clinique, tels que acivicin (Lα-[αS, 5S] amino-3-chloro-4,5-dihydro-5-isoxazoleacétique), DON (6-diazo-5-oxo-L-norleucine), méthionine sulfoximine, etc...

L'action du GAH, qui est un autre analogue de la glutamine, a été étudiée sur plusieurs types de cellules tumorales et normales, aussi bien in vitro que in vivo, ce qui a donné naissance à ce second aspect de la présente invention.

## A - ETUDE IN VITRO : INHIBITION DE LA CROISSANCE DES CELLULES TUMORALES PAR GAH

L'effet du GAH a été étudié sur diverses lignées cellulaires obtenues à partir de tumeurs humaines et fibroblastes de poumon foetal humain (MRC5). Parmi les tumeurs humaines, on a observé un effet inhibiteur total sur la synthèse de l'ADN, aussi bien de tumeurs solides : carcinome de tumeur mammaire (lignée MCF7), lignée de tumeur d' Ewing, mélanome, carcinome du colon (lignée HT29), que de lymphomes : cellules de tumeurs de Burkitt, cellules de lymphome murin RBL5 ou de leucémie L1 210 (Figure 2)

Légende de la Figure 2

- Abscisses : temps de contact des différentes cellules avec le le L-glutamique acide γ monohydroxamate :    1 - L1210
     2 - BL60
     3 - NterDau
     4 - B16

- Ordonnées : incorporation de $^3$H-thymidine (comptage de la radioactivité correspondante en coups par minute (CPM).

Dans ce qui suit, les concentrations du GAH sont en micromoles ou millimoles par litre de milieu cellulaire (ci-après désigné simplement par "μM" ou "mM").

Sur une lignée de mélanome prise comme exemple, on peut voir que l'effet inhibiteur est dépendant de la dose (Figure 3), mais également du temps de contact. Dès 2 heures de contact des cellules de mélanome avec 1,5 mM de GAH, il y a mort cellulaire, qui s'amplifie jusqu'à 72 heures de culture des cellules tumorales, puis les cellules échappent à cette inhibition (Figure 4). L'effet cytolytique total est dépendant de la dose et du temps de contact du GAH avec les cellules tumorales. Ainsi, pour les cellules de mélanome, un contact de 30 heures avec 1,5 mM de GAH est nécessaire pour avoir une mort cellulaire complète à 72 heures, alors que 50 heures de contact sont nécessaires avec une concentration de drogue de 400 μM (Figure 5).

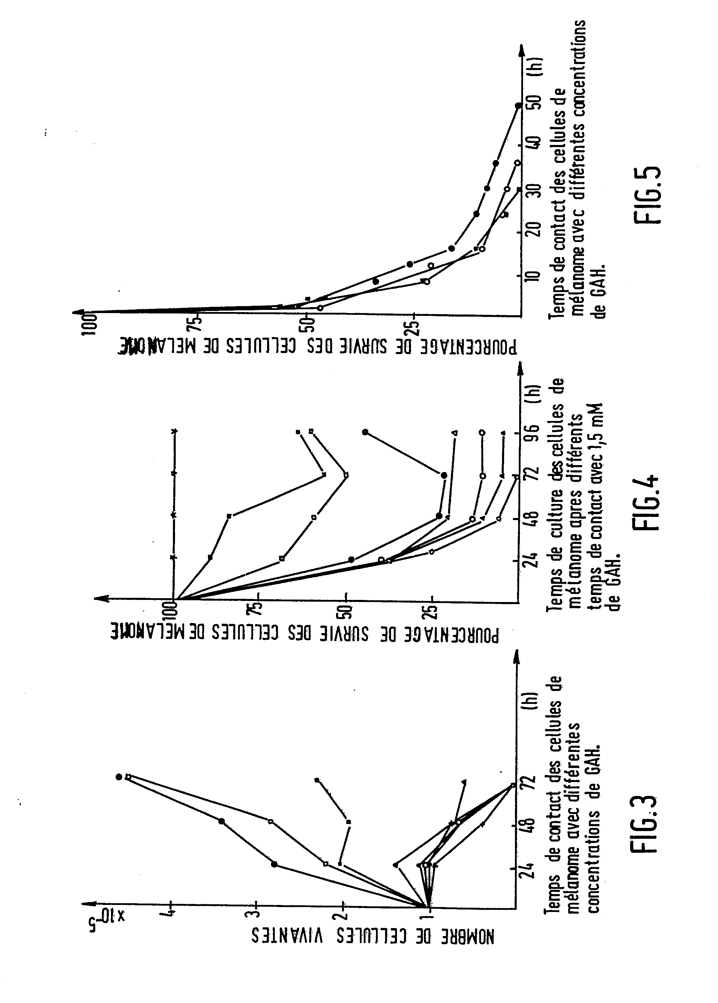

## Légendes des Figures 3 à 5

### Figure 3

- Abscisses : temps de contact des cellules de mélanome avec différentes concentrations de GAH :
● Témoin sans GAH ; cellules de mélanome cultivées avec GAH : □ 25 μM ; ■ 100 μM ; △ 200 μM ; ▲ 400 μM ; ○ 800μM ; ☾ 1 mM ; ☆ 1,5 mM
- Ordonnées : nombre de cellules vivantes.

### Figure 4

- Abscisses : temps de culture des cellules de mélanome après différents temps de contact avec 1,5 mM GAH :
☆ Témoin cellules sans GAH ; Cellules de mélanome incubées avec GAH pendant : ■ 2 heures ; □ 4 heures ; ● 8 heures ; △ 12 heures ; ○ 16 heures ; ▲ 24 heures ; ☆ 30 heures.
- Ordonnées : pourcentage de survie des cellules de mélanome.

### Figure 5 :

- Abscisses : temps de contact des cellules de mélanome avec différentes concentrations de GAH :
● 400 μM ; ○ 1 mM ; ■ 1,5 mM
- Ordonnées : pourcentage de survie des cellules de mélanome à 72 heures.

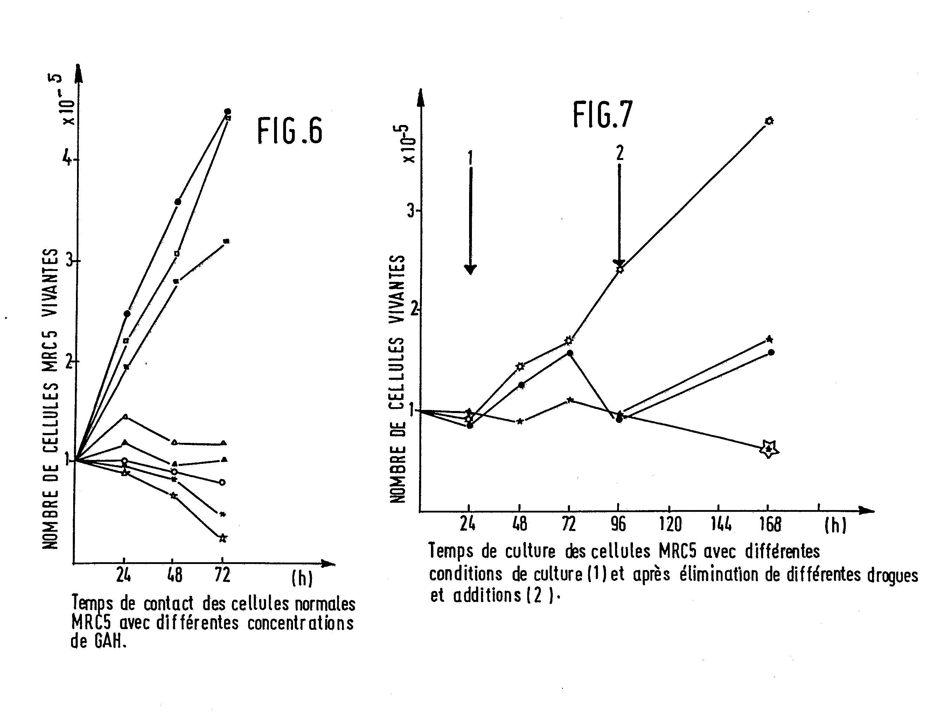

En revanche, le GAH provoque un arrêt de synthèse de l'ADN des cellules normales MRC5, sans que cela s'accompagne de mort cellulaire pour des doses allant jusqu'à 800 μM (figure 6). On observe un effet cytostatique sur les cellules normales qui est réversible après élimination de la drogue. La croissance des cellules MRC5 dans un milieu déprimé en glutamine est aussi arrêtée, avec, cependant, un décalage dans l'inhibition par rapport à l'effet du GAH. La réversion de la croissance des cellules normales cultivées dans un milieu déprimé en glutamine est efficace par addition de glutamine, alors que la croissance de cellules traitées avec le GAH ne reverse pas par addition de glutamine (Figure 7). Il s'agit probablement de deux mécanismes d'action différents.

## Légendes des Figures 6 et 7 :

### Figure 6 :

- Abscisses : temps de contact des cellules normales MRC5 avec différentes concentrations de GAH
● Témoin sans GAH ; cellules MRC5 cultivées avec GAH :
□ 25 μM ; ■ 100 μM ; △ 200 μM ; ▲ 400 μM ; ○ 800 μM ; ● 1 mM ; ☆ 1,5 mM
- Ordonnées : nombre de cellules MRC5 vivantes.

### Figure 7

- Abscisses : temps de culture des cellules MRC5
: 1. avec différentes conditions de culture
○ témoin MRC5 milieu normal
● milieu déprimé en glutamine
● milieu normal avec D-glutamyl acide β (GAH
2. après élimination des différentes drogues et addition
○ milieu normal MRC5 témoin
● milieu avec glutamine 2mM
● milieu normal
☆ milieu GAH + glutamine 2mM
- Ordonnées : nombre de cellules vivantes.

## B - ETUDES IN VIVO SUR MODELES MURINS DE GAH

Compte tenu des résultats in vitro, l'activité anti-tumorale de GAH a été étudiée sur deux modèles tumoraux murins : leucémie L1 210 et mélanome B16.

Pour la leucémie L1 210, maintenue par passage intra-péritonéal chez la souris DBA/2, le nombre de cellules utilisées pour les essais thérapeutiques est de $5.10^5$ cellules par souris.

Pour le mélanome B16, maintenu par passage souscutanés successifs, la dose injectée par voie intra-péritonéale pour les essais thérapeutiques est de $10^6$ cellules par souris.

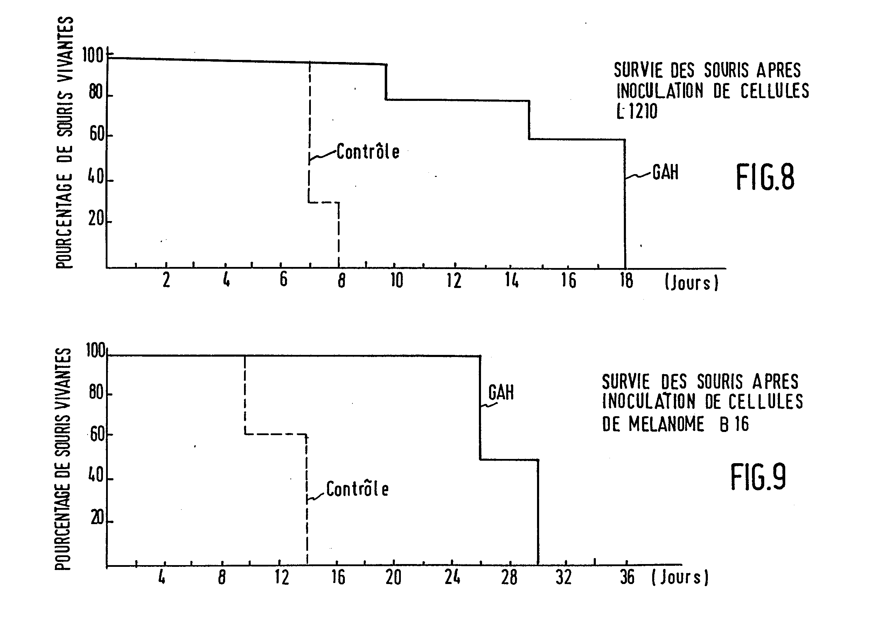

Les résultats positifs qui ont été obtenus sont exprimés par le pourcentage d'augmentation de la survie, considérant comme significative une augmentation de 30%. Les résultats obtenus démontrent une activité antitumorale de GAH (Figures 8 et 9).

## Légendes des Figures 8 et 9

### Figure 8:

Courbe de survie des souris DBA/2-$B_6D_2F_1$ inoculées avec L1 210 ($5 \times 10^5$ cellules dans 0,5 ml IP) après traitement avec L-glutamique acide monohydroxamate : 1200 mg/kg dose/jour, les jours 1, 2, 3, 4 ; et 600 mg/kg dose/jour, les jours 8, 9, 10 (les groupes sont constitués de 8 souris.).
- Abscisses : jours après l'inoculation de cellules tumorales
- Ordonnées : pourcentage de souris vivantes.

### Figure 9:

Courbe de survie des souris C5b16 inoculées avec les cellules de mélanome B16 ($10^6$ cellules dans 0,5 ml IP)après traitement avec GAH : 1200 mg/kg dose/jour, les jours 1, 2, 3, 4 ; 600 mg/kg dose/jour, les jours 8, 9, 10, 11 ; et 300 mg/kg dose/jour, les jours 16, 17, 18, 19 (les groupes traités et non traités sont de 6 animaux).
- Abscisses : jours après l'inoculation de cellules tumorales
- Ordonnées : pourcentage de souris vivantes

En conclusion, le L-glutamique acide γ monohydroxamate montre une activité cytotoxique sur différentes lignées de tumeurs humaines, sans que l'on puisse donner des précisions sur son mécanisme d'action et sur le système enzymatique cible.

La sélectivité d'action (cytolytique et irréversible sur cellules normales) laisse à penser que le GAH utilise une différence métabolique entre cellule tumorale et cellule normale.

La présente invention a également pour objet une nouvelle composition médicamenteuse, destinée notamment au traitement des maladies infectieuses dues aux virus, ainsi qu'au traitement des tumeurs, caractérisée par le fait qu'elle comporte, dans un support pharmaceutiquement acceptable, le L-glutamique acide γ monohydroxamate. La composition de l'invention peut également contenir des additifs inertes ou pharmacodynamiquement actifs.

La composition médicamenteuse de l'invention se présente sous la forme d'une poudre lyophilisée, constituée par le principe actif (GAH), à dissoudre extemporanément dans une solution physiologique, en vue de l'injection. Dans les deux cas (traitement des maladies infectieuses et traitement des tumeurs), le médicament s'administre en effet par voie parentérale par exemple (intraveineuse, intrapéritonéale, dans le liquide céphalo-rachidien etc..). Pour l'injection, le principe actif est dissous à raison de 0,6 à 1,5 g/l dans la solution physiologique.

Par ailleurs, la composition de l'invention peut également contenir des additifs inertes ou pharmacodynamiquement actifs.

L'invention porte également sur l'utilisation de L-glutamique acide γ monohydroxamate pour fabriquer une composition médicamenteuse destinée au traitement des maladies infectieuses dues aux virus, ainsi que sur l'utilisation du L-glutamique acide γ monohydroxamate pour fabriquer une composition médicamenteuse destinée au traitement des tumeurs.

Enfin, l'invention porte sur un procédé de préparation d'une composition pharmaceutique destinée au traitement des maladies infectieuses dues aux virus, caractérisé par le fait qu'on prépare le L-glutamique acide γ monohydroxamate sous une forme de poudre lyophilisée, qui est destinée à être dissoute extemporanément dans une solution physiologique en vue de l'injection, ainsi que sur un procédé de préparation d'une composition pharmaceutique destinée au traitement des tumeurs, caractérisé par le fait qu'on prépare le L-glutamique acide γ monohydroxamate sous une forme de poudre lyophilisée, qui est destinée à être dissoute extemporanément dans une solution physiologique en vue de l'injection.

Il a en outre été observé que le GAH présente un effet cytolytique sur des cellules isolées de métastases, constaté notamment sur les colonies métastasiantes du mélanome B 16 in vitro.

**Revendications**

1 - Médicament, caractérisé par le fait qu'il consiste en le L-glutamique acide γ monohydroxamate.

2 - Médicament selon la revendication 1, caractérisé par le fait qu'il est destiné au traitement des maladies infectieuses dues aux virus.

3 - Médicament selon la revendication 1, caractérisé par le fait qu'il est destiné au traitement des tumeurs.

4 - Composition médicamenteuse, caractérisée par le fait qu'elle renferme, à titre de principe actif, dans un support pharmaceutiquement acceptable, le L-glutamique acide γ monohydroxamate.

5 - Composition médicamenteuse selon la revendication 4, caractérisée par le fait qu'elle est destinée au traitement des maladies infectieuses dues aux virus.

6 - Composition médicamenteuse selon la revendication 4, caractérisée par le fait qu'elle est destinée au traitement des tumeurs.

7 - Composition médicamenteuse selon l'une des revendications 4 à 6, caractérisée par le fait qu'elle se présente sous la forme d'une poudre lyophilisée constituée par le principe actif, à dissoudre extemporanément dans une solution physiologique, en vue de l'injection.

8 - Composition médicamenteuse selon la revendication 7, caractérisée par le fait que le principe actif est dissous à raison de 0,6 à 1,2 g/l de la solution physiologique.

9 - Composition selon l'une des revendications 4 à 7, caractérisée par le fait qu'elle contient des additifs inertes ou pharmacodynamiquement actifs.

10 - Utilisation du L-glutamique acide γ monohydroxamate pour fabriquer une composition médicamenteuse destinée au traitement des maladies infectieuses dues aux virus.

11 - Utilisation du L-glutamique acide γ monohydroxamate pour fabriquer une composition médicamenteuse destinée au traitement des tumeurs.

12 - Procédé de préparation d'une composition pharmaceutique destinée au traitement des maladies infectieuses dues aux virus, caractérisé par le fait qu'on prépare le L-glutamique acide γ monohydroxamate sous une forme de poudre lyophilisée, qui est destinée à être dissoute extemporanément dans une solution physiologique en vue de l'injection.

13 - Procédé de préparation d'une composition pharmaceutique destinée au traitement des tumeurs, caractérisé par le fait qu'on prépare le L-glutamique acide γ monohydroxamate sous une forme de poudre lyophilisée, qui est destinée à être dissoute extemporanément dans une solution physiologique en vue de l'injection.

0253697

FIG.1

LOG D'INHIBITION DE LA FORMATION DE PFU /ml

µM

FIG.2

CPM 25.10⁴

INCORPORATION DE ³H-THYMIDINE

20.10⁴
15.10⁴
10.10⁴
5.10⁴

1
2
3
4
Contrôle

24    48    72    (heures)

TEMPS DE CONTACT DES DIFFERENTES CELLULES AVEC GAH

# FIG.1A

# FIG.1B

Temps de contact des cellules de mélanome avec différentes concentrations de GAH.

FIG.3

Temps de culture des cellules de mélanome apres différents temps de contact avec 1,5 mM de GAH.

FIG.4

Temps de contact des cellules de mélanome avec différentes concentrations de GAH.

FIG.5

**FIG.6**

NOMBRE DE CELLULES MRC 5 VIVANTES

$\times 10^{-5}$

Temps de contact des cellules normales MRC5 avec différentes concentrations de GAH.

**FIG.7**

NOMBRE DE CELLULES VIVANTES

$\times 10^{-5}$

Temps de culture des cellules MRC5 avec différentes conditions de culture (1) et après élimination de différentes drogues et additions (2).

0253697

SURVIE DES SOURIS APRES
INOCULATION DE CELLULES
L·1210

FIG.8

SURVIE DES SOURIS APRES
INOCULATION DE CELLULES
DE MELANOME  B 16

FIG.9